# EUROPEAN PATENT SPECIFICATION

(11) **EP 0 256 553 B1**
(45) Date of publication and mention of the grant of the patent: **20.07.1994**
(21) Application number: 87111974.9
(22) Date of filing: 18.08.1987
(51) Int. Cl.: C12N 15/00, C12N 1/20, C12P 21/02

(54) **Gene encoding insecticidal protein and production of said protein using said gene**
Gen, das ein Insektizid-Protein kodiert, und Herstellung des Proteins durch Verwendung dieses Gens
Gène codant une protéine insecticide et production de ladite protéine à l'aide de ce gène

(30) Priority: 19.08.1986 JP 193483/86
(43) Date of publication of application: 24.02.1988
(73) Proprietor: SUMITOMO CHEMICAL COMPANY, LIMITED, Chuo-ku Osaka 541 (JP)
(72) Inventor: Oeda, Kenji, Toyonaka-shi Osaka-fu (JP); Oshie, Kazuyuki, Toyonaka-shi Osaka-fu (JP); Shimizu, Masatoshi, Ashiya-shi Hyogo-ken (JP); Nakamura, Keiko, Kobe-shi Hyogo-ken (JP); Ohkawa, Hideo, Ashiya-shi Hyogo-ken (JP)
(74) Representative: VOSSIUS & PARTNER

(56) References cited:
- EP-A- 0 063 949
- EP-A- 0 224 331
- MOL. BIOL. MICROB. DIFFER. PROC. INTERNAT. CONF. 9th, 1984, (publ. 1985), pages 217-224; A. KLIER et al.: "Cloning and expression in Escherichia coli of the crystal protein gene from Bacillus thuringiensis strain aizawa 7-29 and comparision of the structural organization of genes from different serotypes"
- THE JOURNAL OF BIOLOGICAL CHEMISTRY, vol. 260, no. 10, 25th May 1985, pages 6264-6272, The American Society of Biological Chemists, Inc., US; H. ERNEST SCHNEPF et al.: "The amino acid sequence of a crystal protein from Bacillus thuringiensis deduced from the DNA base sequence"
- GENE, vol. 52, no. 2-3, 1987, pages 285-290, Elsevier Science Publishers B.V., Amsterdam, NL; M.Z. HAIDER et al.: "Cloning and heterologous expression of an insecticidal delta-endotoxin gene from Bacillus thuringiensis var. aizawai IC1 toxic to both lepidoptera and diptera"
- Entomophaga, 30 (2), 1985, pp.177-186.
- Microbial Control of Pests and Plant Diseases 1970-1980, ed. H.D. Burges, Academic Press, London 1981 (page 194, first paragraph).

## Description

The present invention relates to a gene coding for an insecticidal protein produced by Bacillus thuringiensis aizawai a7A021 and a process for the production of the insecticidal protein by the use of the gene, said protein being capable of killing injurious insects such as Plutella xylostella and Spodoptera litura which belong to an order Lepidoptera. The invention also relates to an expression plasmid containing the gene and to a microorganism which has been transformed with the expression plasmid.

### Background of the invention

It is known that a strain belonging to Bacillus thuringiensis produces, at a sporulation phase, a crystal of 1-2»m in size of the insecticidal protein, and that intake of the crystal by insects leads to the stoppage of feeding behavior, enterorrhexis, and death of the insects. Bacillus thuringiensis strains are classified into 29 subspecies according to flagellar antigens and esterase activities they produce, and each strain exhibits specific insecticidal activity.

The cloning and expression of a crystal protein gene of a 45 Md host plasmid from Bacillus thuringiensis aizawai 7-29 and the sequence of another crystal protein from a Bacillus thuringiensis var. kurstaki HD-1-Dipel plasmid has been described by Klier et al. (Mol. Biol. Microb. Differ. Proc. Internat. Conf. 9th, 1984 (publ. 1985), pages 217-224) and Schnepf et al. (J. Biol. Chem. 260 (1985), pages 6264-6272), respectively. Furthermore, EP-A1 224 331 discloses the cloning and expression of an insecticidal protein gene from Bacillus thuringiensis subsp. aizawai IPL No. 7 harbouring 12 different plasmids.

The inventors have found that an insecticidal protein produced by Bacillus thuringiensis aizawai a7A021 strain exhibits an intense insecticidal activity on Plutella xylostella and Spodontera litura. Subsequently, the inventors have succeeded in cloning a chromosomal gene which encodes the insecticidal protein and sequencing a full length of the structural gene comprising 3528 base pairs. On the basis of these successful works, the inventors have also succeeded in the production of a substantial amount of the insecticidal protein by inserting the gene coding for the protein into an expression vector, transforming a microorganism with the resultant plasmid, and culturing the transformed microorganism under appropriate conditions. In addition, the inventors have determined the primary structure of the insecticidal protein.

Accordingly, the invention provides a gene coding for an insecticidal protein produced by Bacillus thuringiensis aizawai a7A021 strain. The invention also provides an expression plasmid which carries the gene integrated therein, and a host cell transformed with the expression plasmid. The invention further provides a process for the production of the insecticidal protein which comprises culturing the transformed microorganism under appropriate conditions.

In the accompanying drawings:
Fig.1 is a restriction map of foreign DNA (3.5kb) inserted in plasmid pCA1 and pCA5, said foreign DNA encoding insecticidal protein of Bacillus thuringiensis aizawai a7A021. The symbols Ps, C, E, Pv, S, H, K, and B respectively represent restriction enzymes Pst I, Cla I, Eco RI, Pvu II, Sal I, Hind III, Kpn I, and Bam HI.
Fig.2 shows the base sequence of a full length of structural gene (3528 bp) for insecticidal protein in upper line and the corresponding amino acid sequence in the lower.
Fig.3(A) and (B) respectively show densitometric determination of insecticidal protein found in crude extracts of E. coli JM103/pKC6 culture and E. coli JM103/pKK223-4 culture (control). The arrow indicates the position of insecticidal protein.
Fig.4 shows the construction of expression plasmid pKC6. Hatching box represents the gene encoding insecticidal protein. Tac means Tac promoter. rrnB[T] means rrnB terminator. The symbols Kp, Pv, Bm and Ps represent restriction enzymes Kpn I, Pvu II, Bam HI and Pst I, respectively.

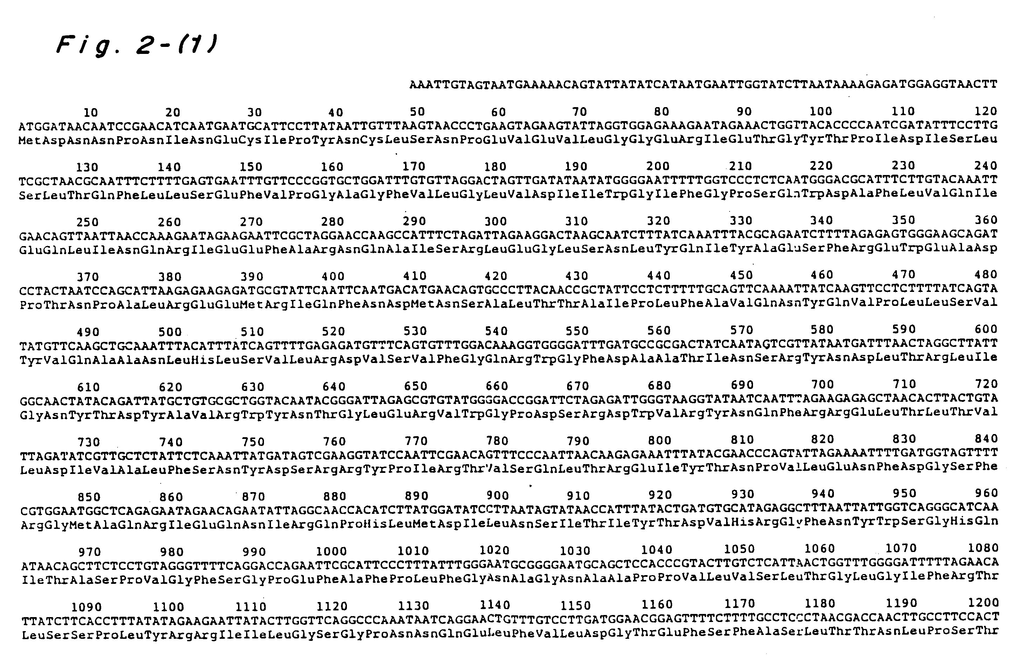

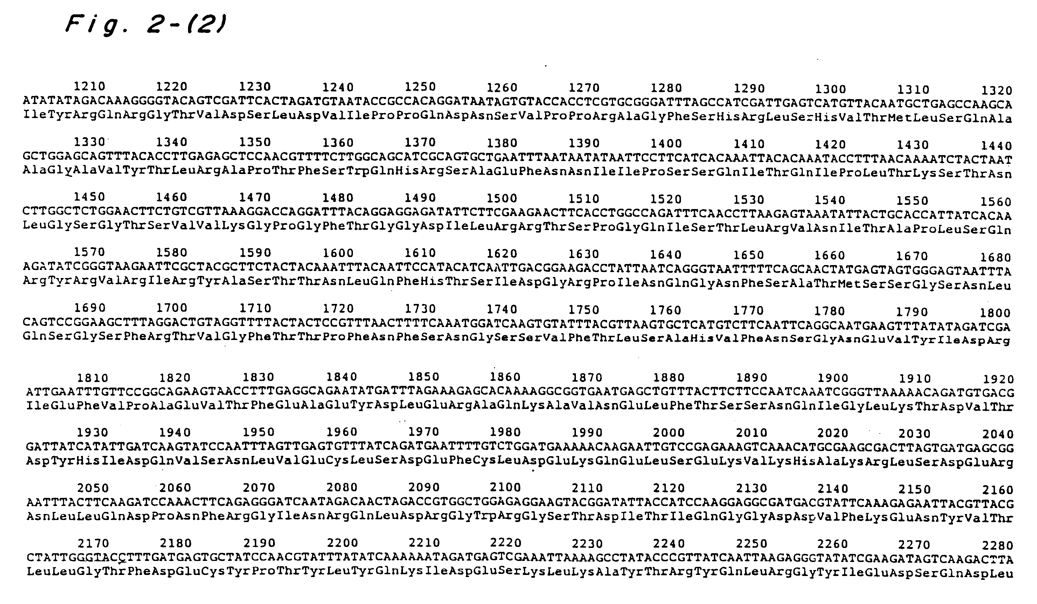

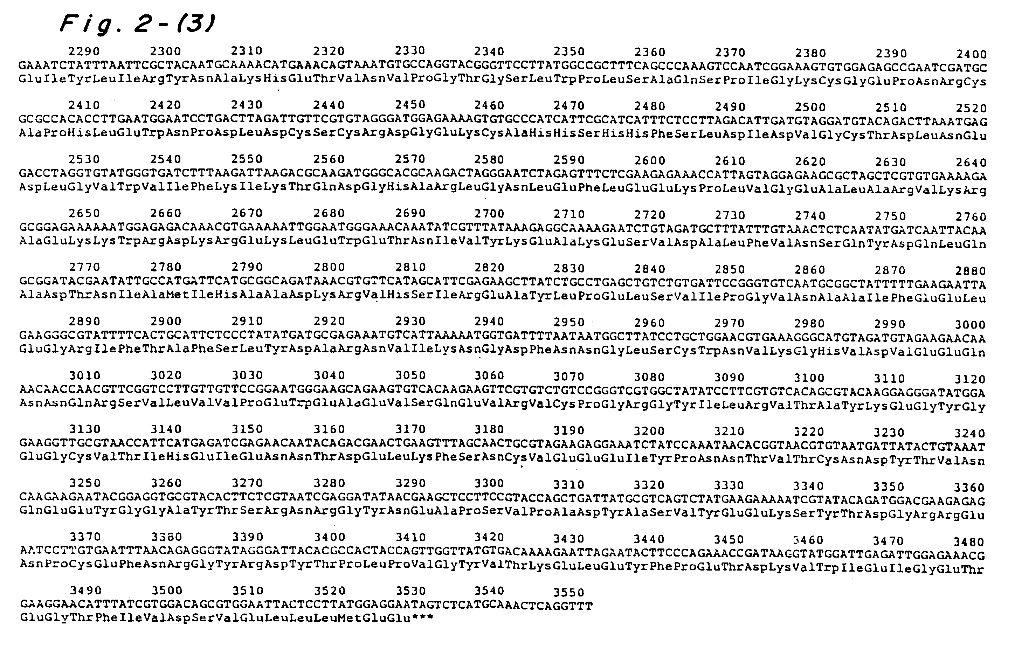

The gene encoding the insecticidal protein produced by Bacillus thuringiensis aizawai a7A021 strain, which is characterized by the base sequence and amino acid sequence shown in Fig.2 of the accompanying drawings, is harbored in E. coli JM103/pKC6 (ATCC 67487) and can be obtained from the E. coli JM103/pKC6 or by screening a gene library prepared from the chromosomal DNA of the strain by the use of a suitable probe which has been synthesized on the basis of the base sequence given in Fig. 2. The probe can also be prepared on the basis of the known base sequence of the gene encoding an insecticidal protein produced by an analogous strain, Bacillus thuringiensis kurstaki HD-1 Dipel.

As well known to the skilled in the art, there exist a plurality of codons for each amino acid with some exceptions and, therefore, a variety of base sequences or DNA sequences are possible for a given amino acid sequence. Accordingly, the gene of the present invention, which encodes the insecticidal protein, is not limited to the natural gene represented by the DNA sequence depicted in Fig. 2. In other words, the gene of the invention should be construed to include every gene which may be naturally or artificially derived and codes for the amino acid sequence shown in Fig. 2. On the other hand, a mutant of the natural gene which codes for an analogous amino acid sequence closely related to the one given in Fig. 2 can be prepared by effecting insertion, deletion, and/or substitution of a nucleotide or nucleotides on the natural gene. Such mutation can be conventionally conducted either without changing the characteristic property of the natural protein encoded by the natural gene, or being accompanied by the improvement of the characteristics. Such mutated genes obtainable by the above procedures are also included in the term "a gene encoding insecticidal protein" of the present invention.

The expression plasmid capable of expressing the insecticidal protein in microorganism cells can be constructed by inserting the gene of the invention into an expression vector. Suitable vectors include plasmid pUC18 carrying lac promoter (available from Pharmacia), plasmid pKK223-4 carrying Tac promoter, which is a strong promoter constructed from Escherichia coli plus a terminator of rrnB ribosomal RNA, plasmid pDR720 carrying Trp promoter (available from Pharmacia), and an inducible plasmid pPL-lamda (available from Pharmacia).

Incorporation into a host microorganism, for instance, E. coli JM103 (available from Pharmacia), of the resultant expression plasmid bearing the gene for the insecticidal protein provides a transformed microorganism which produces the protein, and cultivation of the transformant under appropriate conditions yields a substantial amount of the insecticidal protein. The cultivation may be conducted, for example, at 37°C for 8 to 20 hours with shaking, in a culture medium comprising 1 litre of distilled water, 10g of tryptone, 5g of NaCl, and 5g of yeast extract, at pH 7.5.

After completion of the cultivation, the culture is homogenized through, for instance, ultrasonic wave and centrifuged to give the insecticidal protein in the form of condensates.

Various host-vector systems derived from Bacillus, Saccharomyces, Pseudomonas, and Actinomyces can be employed in place of those of E. coli.

The following examples further illustrate and describe the invention disclosed herein. The invention is not limited in scope by reason of the following Example.

The reagents and materials employed in the following Example are available from the following providers: tryptone, yeast extract, starch agar, and salmon sperm-single-stranded DNA.....Sigma; NaCl, dithiothreitol, and adenosine triphosphate.....Nakarai Chemical; E. coli polynucleotide kinase, E. coli alkaline phosphatase, T₄ DNA ligase, Klenow buffer, Klenow fragment enzyme, CHASE solution, dNTP, ddNTP, and restriction enzyme Bam HI......Takara shuzo; [γ-³²P]ATP.....Amersham Japan; DE-52 column..... Whattman; restriction enzyme Aha III, pUC18 vector plasmid, and expression vector pKK223-3.....Pharmacia; low melting point agarose gel.....Bethesda Research Laboratory; primer DNA.... PL Biochemical.

### Example 1

A.  Screening of Bacillus thuringiensis aizawai a7A021

Bacillus thuringiensis aizawai IPL No. 7, a strain deposited under the Budapest Treaty at the Fermentation Research Institute in Japan under the accession number FERM BP-1150, was cultured overnight at 30°C in PY liquid medium (10g tryptone, 5g NaCl, 5g yeast extract, pH 7.0, total volume: 1 l) containing acridine orange (0.04 »g/ml). A 10⁵ dilution (0.1ml) of the culture was inoculated on a starch-agar plate, which had been obtained by dissolving 25g of starch agar in 1 litre of distilled water followed by autoclaving, and cultured at 30°C for three days, and desired colonies were selected in the following manner: the spore and crystal were observed under a phase-contrast microscope, and plasmid DNA was prepared by alkaline rapid method and analyzed by agarose gel electrophoresis. These manipulations gave a7A021 strain which lost a 52 Md plasmid containing a gene encoding other insecticidal protein.

After the a7A021 strain was cultured in PY liquid medium at 30°C for seven days, the cells were collected, subjected to freeze-thaw treatment three times, suspended in distilled water, and homogenized by ultrasonic wave to yield a crystal suspension. A leaf of Cymbidium Kanran was dipped in an aliquot of the suspension containing 2.2x10⁷ spores per ml or a diluted aliquot containing 2.2x10⁶ spores per ml. Subsequently, 3-instar larvae of Plutella xylostella were allowed to feed the leaf.

The test revealed that the suspension of a concentration of 2.2x10⁷ spores/ml killed 20 larvae among 20 larvae tested, and 2.2x10⁶ concentration killed 12 larvae among 20. Similar test was conducted on 4-instar larvae of Spodoptera litura, and a concentration of 2.6x10⁸ spores/ml killed seven larvae among 20.

These test results revealed that Bacillus thuringiensis aizawai a7A021 possessed strong insecticidal activity on both injurious insects mentioned above.

B.  Cloning of the gene encoding insecticidal protein
    produced by Bacillus thuringiensis aizawai a7A021

### Step 1 Preparation of synthetic DNA probe

Synthetic DNA probes, 5ʹ-CACAAATCCAGCACCGGG-3ʹ and 5ʹ-CCTCCATAAGGAGTATTT-3ʹ were prepared on the basis of the nucleotide sequence of the gene encoding the insecticidal protein produced by Bacillus thuringiensis kurstaki HD-1 Dipel (Whiteley et al., J. Biol. Chem., 260, 6264-72, 1985).

The DNA oligomers were prepared using DNA Synthesizer Model 380A (Applied Biosystem Co.). A 27% NH₄OH solution (1ml) was added to a DNA collector, which was then warmed at 55°C for 4 hours. After the solution was concentrated to dryness, the residue was dissolved in 100»l of 0.01M triethylamine-acetic acid (TEAA) (pH 7.2). The mixture was injected into a reverse phase HPLC column C18 and eluted with a solvent system comprising acetonitrile-0.1M TEAA (pH 7.2). The fractions having absorption peak at 260nm were collected and evaporated to dryness. The residue, after added with 100»l of acetonitril-80% acetic acid, was left to stand for 15 minutes, whereby the mixture turned to pale orange color. After the change of color, the mixture was evaporated to dryness, and admixed with 100»l of 0.01M TEAA (pH 7.2) and then 100»l of ethyl acetate. The ethyl acetate layer was discarded, and diethyl ether was added to the residue. After this manipulation was repeated twice, the mixture was evaporated to dryness. The residue was dissolved in 0.01M TEAA (pH 7.2) and subjected to reverse phase HPLC. Fractions having absorption peak at 260nm were collected, evaporated to dryness, and dissolved in a 10mM Tris-HCl (pH 7.5) + 1mM EDTA solution (TE solution).

The resultant synthetic DNA probes were ³²P-labelled as follows. To 5»l of synthetic DNA probe (ca. 100pmole) were added 15 units of E. coli polynucleotide kinase, 100»Ci of [γ-³²P]ATP, and 10»l of 10 x reaction mix (0.5M Tris-HCl, pH 7.6, 0.1M MgCl₂, 0.1M 2-mercaptoethyl alcohol), and thereafter, added distilled water to a total volume of 100»l. The mixture was allowed to react at 37°C for one hour, added with chloroform and phenol (1:1) with agitation, and the supernatant was separated. The supernatant was applied to DE-52 column (bed size: 0.5ml, Whattman) which had been equilibrated with TE buffer (pH 7.5). After washing with 3ml of TE buffer (pH 7.5), the column was eluted with 0.7M NaCl-TE buffer (pH 7.5), whereby radioactivefractions were separated to provide ³²P-labelled synthetic DNA probe.

### Step 2 Preparation of chromosomal DNA library of Bacillus thuringiensis aizawai a7A021

Bacillus thuringiensis aizawai a7A021 was inoculated in 250ml of PY liquid medium and cultured overnight. The cells were collected by centrifuging the culture at 6,000 rpm for 10 minutes and washed with 100ml of 100 mM NaCl, 10mM Tris-HCl (pH 7.9)-10mM EDTA. The cells were collected again, suspended in 10ml of 150mM NaCl-110mM EDTA (pH 7.9), and added with lysozyme at a final concentration of 0.25mg/ml. After the mixture was incubated at 37°C for one hour, 13ml of 100mM Tris-HCl (pH 7.9)-100mM NaCl-2% SDS was added thereto with gentle agitation. The resultant mixture was extracted four times with a phenol-chloroform (1:1) mixture saturated with just-mentioned SDS solution, and the upper layer was separated. To the layer was added 2 volumes of cold ethanol, and the resulting white precipitate was recovered using a glass stick. The precipitate was washed three times with 80% ethanol, dried and suspended in 200»l of distilled water.

The resultant total DNAs (10»g) were digested with restriction enzyme Aha III (10 Units) at 37°C for one hour in 30»l of an Aha III reaction mix (10mM Tris-HCl, pH 7.5, 60mM NaCl, 7mM MgCl₂, 10mM EDTA, 1mM dithiothreitol), and the resultant mixture was subjected to agarose gel electrophoresis using 0.8% low melting point agarose gel containing 0.1»g/ml of ethidium bromide. A band which contained 3.5kb Aha III-digested DNA fragments was excised from the gel under UV lamp, and the excised gel was heated at 65°C for 5 minutes in a test tube. To the melted gel was added two volumes of TE buffer (10mM Tris-HCl, pH 8.0, 0.5mM EDTA), and the mixture was extracted with phenol saturated with TE buffer (10mM Tris-HCl, pH 8.0, 1mM EDTA). After 5 minutes centrifugation at 10,000 rpm (7000 g), the upper layer was separated, added with 1/40 volume of 4M NaCl plus 2 volumes of ethanol, and left to stand for 10 minutes at -80°C to precipitate DNA. The DNA was recovered after 10 minutes centrifugation (10,000rpm, 7000 g) and suspended in 10»l of distilled water.

On the other hand, 5»g of pUC18 plasmid was digested with 5 units of restriction enzyme Hinc II. Phenol saturated with the same volume of TE buffer was added thereto with agitation, and the upper layer was separated. The DNA was precipitated with ethanol and suspended in 20»l of distilled water. To the suspension were added 5»l of E. coli alkaline phosphatase (2 units), 50 »l of 0.1M Tris-HCl buffer (pH 80), and 1.5»l of distilled water, and the mixture was incubated at 60°C for one hour and treated with alkaline phosphatase. Subsequently, the mixture was treated twice with phenol/chroloform, and the upper layer was separated. DNA was precipitated by addition of ethanol, and the precipitate was suspended in distilled water.

The Aha III-digested DNA (10»l) and the Hinc II -digested pUC 18 vector DNA (10»l) were combined. To the mixture were added 1»l of T₄DNA ligase (0.1unit), 7.5»l of 0.1M dithiothreitol, 7.5»l of 10mM adenosine triphosphate, 25»l of 3 x reaction mix (200mM Tris-HCl, pH 7.6, 20mM MgCl₂), and 5 »l of distilled water to the total volume of 75»l, and the resultant mixture was incubated at 14°C for 6 hours. The reaction mixture (10»l) was combined with 100»l of competent cells (100»l) of E. coli DH1 (ATCC 33849), which had been prepared according to the teaching of Scott, Cell Technology, vol. 2, pp 616-626, 1983, and the combined mixture was incubated at 0°C for 15 minutes. After heat shock at 42°C for 40 seconds, 0.9ml of L broth liquid medium, which had been prepared by adding 10g tryptone, 5g NaCl, 5g yeast extract, 1g glucose, and distilled water to make a total volume of 1 litre, was added thereto. The mixture was incubated at 37°C for one hour and plated on L broth agar plate which had been prepared by adding agar to L broth liquid medium to make 1.2% concentration, containing a final concentration of 50»g/ml of ampicillin..

### Step 3 Identification and isolation of the clone encoding insecticidal protein by colony hybridization

Colonies on the plate were replicated onto two nitrocellulose filters. The colonies were also transferred to an agar plate containing 50»g/ml ampicillin and 600»g/ml chloramphenicol, and subsequently incubated overnight.

Each of the filters was treated with 2.5ml of 0.5N NaOH for five minutes, air dried, and left to stand for five minutes after addition of the same volume of 1M Tris-HCl, pH 7.5. The filter was air dried, treated with 2.5ml of 1M Tris-HCl, pH 7.5, plus 1.5M NaCl for five minutes, air dried again, and kept at 80°C in vacuo for three hours. Each four filters was treated with 10/4mM/l of 0.1% SDS-4xSCC at 60°C for 15 minutes, said SCC consisting of 0.15M NaCl-0.015M sodium citrate, pH 7.5. After wiping the colonies away from the filters, the latter was immersed at 60°C for three hours in 6ml of pre-hybridizing solution consisting of 4 x SCC, 10 x Denhardt solution, and 100»g/ml salmon testis single-stranded DNA, the 10 x Denhardt solution consisting of 0.2% Ficoll, 0.2% polyvinylpyrrolidone, and 0.2% BSA. Subsequently, the filters were immersed in a hybridizing solution which consisted of the just mentioned pre-hybridizing solution and the ³²P-labelled DNA probe prepared in Step 1, and incubated overnight at 56°C. After hybridization, the filters were rinsed for 15 minutes four times at 56°C with a 4xSCC-0.1% SDS solution, air dried, interposed between X-ray films, and subjected to autoradiography. The colonies which gave positive signals were selected from the master plates. The similar colony-hydridization was repeatedly conducted to isolate positive clones of E. coli DH1/pCA5 and DH1/pCA1.

### Step 4 Analysis of the gene encoding insecticidal protein produced by aizawai a7A021 strain

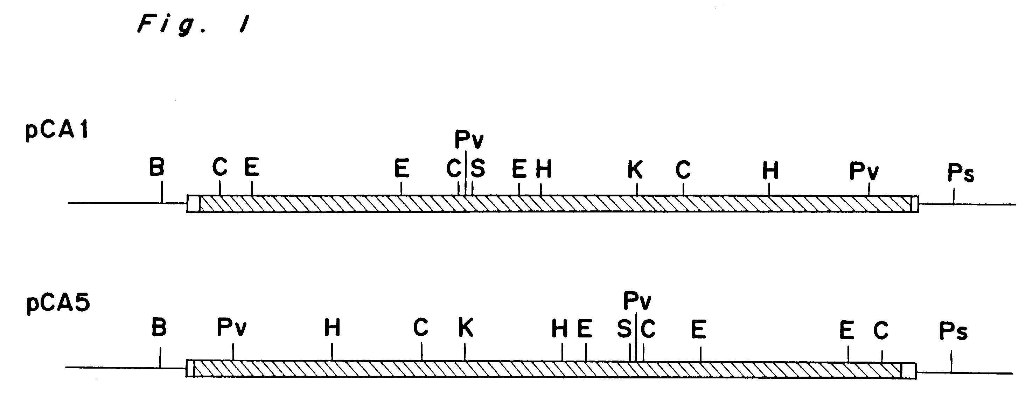

Plasmids pCA5 and pCA1 were respectively isolated from the positive clones E. coli DH1/pCA5 and DH1/pCA1 in accordance with the teaching of Birnbiom and Doly (Nucl. Acids. Res. 7 (1979) 1513-1523). The plasmids pCA5 and pCA1 were each digested with restriction enzymes Cla I, Eco RI, Sac I, Hind III, and Kpn I, and analyzed by 0.7% agarose gel electrophoresis. A restriction map of the inserted DNA (ca. 3.5kb) is presented in Fig. 1 of the accompanying drawings. The analysis of the DNA revealed that the plasmids pCA1 and pCA5 differ only with respect to the orientation of the inserted DNA.

For the purpose of determination of the base sequence, plasmid pCA5 was digested with a variety of restriction enzymes, and the resulting fragments were subcloned into vector plasmids pUC8 and pUC9 in accordance with the method disclosed by Birnboim and Doly (Nucl. Acids. Res. 7 (1979) 1513-23). The resultant plasmid DNAs were suspended in 18»l of TE buffer (pH 7.5), added with 2»l of 2N NaOH, and allowed to stand at room temperature for five minutes. After addition of 8»l of 7.5M ammonium acetate, the DNAs were precipitated by the addition of 100»l of cold ethanol. The mixture was centrifuged at 12,000 rpm (7000 g) for five minutes to recover the precipitate, which was subsequently dried and dissolved in distilled water at a concentration of 0.5pmol/5ml.

To the DNAs obtained above (5pmol) were added 1.5»l of 10 x Klenow buffer, 1»l of primer DNA, and 4.5»l of distilled water to a total volume of 12»l, and the mixture was warmed at 60°C for 15 minutes and allowed to stand at room temperature for 20 minutes.

The above reaction mixture was combined with 2»l of [α-³²P]ATP (400Ci/mmol) and 1»l of Klenow fragment enzyme (2 units). Each of four 3.2»l portions of the resultant mixture was added to one of four kinds of dNTP+ddNTP mixtures (2»l), and the resultant solutions were allowed to stand at 42°C for 20 minutes. Each solution was added with 1»l of CHASE solution, allowed to stand at 42°C for 20 minutes, and finally combined with 6»l of 95% formamide dye which comprises 0.1% Bromophenol Blue and 0.1% xylene cyanol. Each solution (2»l) thus obtained was applied to 6% acrylamide/urea gel prepared in conventional manner and subjected to electrophoresis at 1,700V for six hours. The gel was dried, interposed between X-ray films, and exposed, and the base sequence of the DNA was determined (Fig.2).

As shown in the figure, the gene coding for the insecticidal protein produced by Baccillus thuringiensis aizawai a7A021 comprises the coding region consisting of 3,531 base pairs including the starting codon ATG and the stop codon TAG. The gene encodes 1176 amino acids.

C. Production in E. coli of insecticidal protein naturally
produced by Bacillus thuringiensis aizawai a7A021

### (1) Production of insecticidal protein by E. coli JM 103/pCA1 and JM103/pCA5

Plasmids pCA5 and pCA1 were isolated respectively from the aforementioned positive clones DH1/pCA5 and DH1/pCA1 and transformed into E. coli JM103 in a conventional manner to yield transformants E. coli JM103/pCA5 and JM103/pCA1, respectively.

E. coli JM103/pCA5 and JM103/pCA1 were cultured overnight in L broth liquid medium, and 0.1ml of the overnight culture was transplanted to 10ml of L broth liquid medium, which was subsequently cultured at 37°C for 15 hours. An aliquot (0.3ml) of the culture was centrifuged at 3,000 rpm for 15 minutes to collect the cells, which was then suspended in 50»l of a sample buffer (62.5mM Tris-HCl, pH 8.0, 2% (w/w) sodium dodecyl sulfate, 5% (v/v) 2-mercaptoethyl alcohol, 10% (w/w) glycerol, 0.01% Blomophenol Blue). The suspension was heated at 100°C for five minutes and subjected to SDS-polyacrylamide gel electrophoresis according to the method of Lasemmli et al., Nature 227, 680-685. After completion of the electrophoresis, the gel was stained with Coomassie Brilliant Blue, dried, and fixed to a filter paper.

E. coli JM103/pCA1 was manipulated in the same manner as disclosed above.

A band containing the insecticidal protein having a molecular weight of 130KDal was detected in both E. coli JM103/pCA5 and JM103/pCA1. The band did cross-react with an antibody (IgG) to the insecticidal protein. Quantitative analysis of the band by a densitometer revealed that E. coli JM103/pCA1 and JM103/pCA5 respectively produced 1% and 8% of the insecticidal protein per total proteins. Seventy two base pairs of the 5ʹ untranslated region of the inserted DNA were supposed to contain a promoter functionable in E. coli.

### (2) Production of insecticidal protein by E. coli JM 103/pKC6.

### Step 1 Preparation of plasmid vector

About 5»g of expression vector pKK 223-3 was digested with restriction enzyme Bam HI (0.1 unit) at 37°C for 15 minutes in a Bam HI reaction mix (10mM Tris-HCl, pH 8.0, 7mM MgCl₂, 100mM NaCl, 2mM 2-mercaptoethyl alcohol, 0.01% bovine serum albumin). The resultant mixture was subjected to agarose gel electrophoresis by the use of 0.8% low melting point agarose gel which contained 0.1»g/ml of ethidium bromide. A band containing DNA molecule (4.6kb) which was supposed to have been generated by partial digestion of one of two restriction recognition sites in pKK223-3 was excised under UV lamp, introduced into a test tube, and heated at 65°C for five minutes. The gel was melted, extracted with phenol, and precipitated with ethanol. The recovered DNA was suspended in 20»l of distilled water. To the DNA (∼1»g) was added T₄ DNA ligase (3 units), and the mixture was allowed to react at 16°C for two hours in 45»l of a ligase reaction mix (66mM Tris-HCl, pH 7.6 6.6mM MgCl₂, 10mM dithiothreitol, 1mM ATP). Subsequently, E. coli JM103 was transformed with the resulting solution according to the method disclosed by Cohen (Proc. Natl. Acad. Sci. USA, 69, 2110-2114). The resultant colonies resistant to ampicillin were cultured for the preparation of plasmid DNAs according to the method disclosed by Birnboim and Doly (Nucl. Acids. Res. 7 (1979) 1513-23). About 1»g of the plasmid DNAs was digested with restriction enzyme Bam HI (3 units) at 37°C for one hour in a Bam HI reaction mix, and subjected to agarose gel electrophoresis. A plasmid, which retained Bam HI site for multicloning and lost the other Bam HI site presented in plasmid pKK 223-3, was selected and designated pKK 223-4. The pKK223-4 DNA (∼5»g) was digested with restriction enzymes Pst I (10 units) and Bam HI (10 units) at 37°C for one hour in 50»l of Pst I reaction mix (10mM Tris-HCl, pH 7.5, 50mM NaCl, 10mM MgCl₂, 1mM 2-mercaptoethyl alcohol, 100»g/ml bovine serum albumin). After phenol extraction and ethanol precipitation, the DNA was recovered and suspended in 20»l of distilled water.

### Step 2 Preparation of Pst I - Bam HI fragment comprising the gene encoding insecticidal protein

About 5»g of expression plasmid pCA5, which comprised the gene encoding the insecticidal protein, was digested with about 20 units of Pst I and about 20 units of Bam HI at 27°C for one hour in 50»l of Pst I reaction mix (10mM Tris-HCl, pH 7.5, 50mM NaCl, 10mM MgCl₂, 1mM 2-mercaptoethyl alcohol, 100»g/ml bovine serum albumin), and the resulting mixture was combined with 30»l of phenol saturated with TE buffer. The upper layer was separated after centrifugation at 10,000 rpm (7000 g) for five minutes, added with 1/40 volume of 4M NaCl and two volumes of ethanol, and allowed to stand at -80°C for 10 minutes. The DNA was recovered by centrifugation at 10,000 rpm (7000 g) for 10 minutes, dried, and suspended in 20»l of distilled water.

### Step 3 Construction of Expression plasmid pKC6

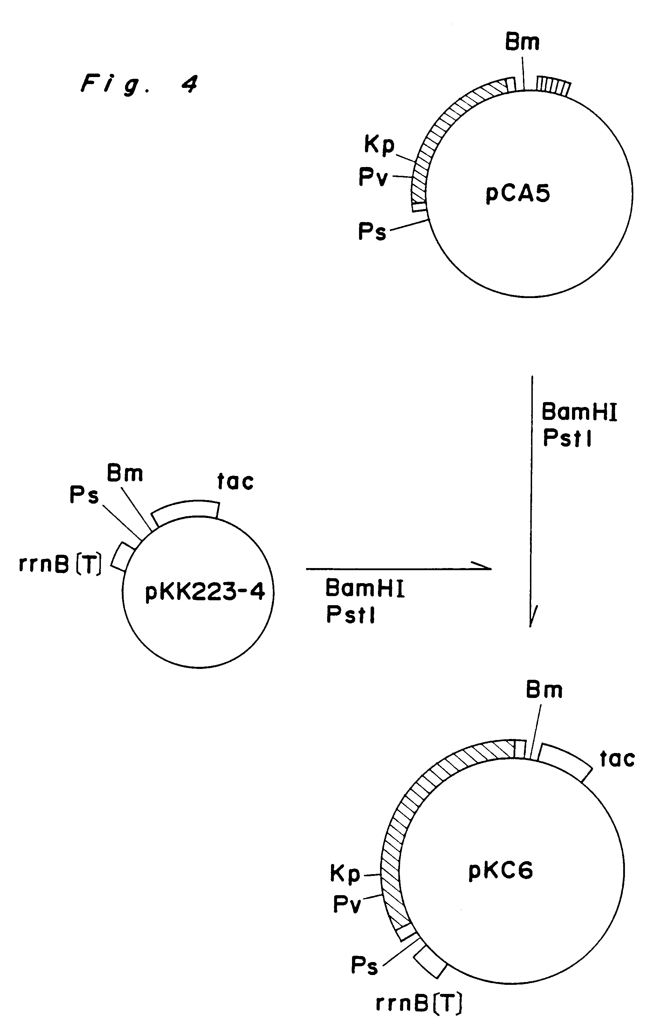

Each 1»g of the Pst I - Bam HI digested vector pKK223-4 derived from Step 1 and Pst I - Bam HI fragment derived from Step 2 were combined. The mixture was allowed to react at 16°C for two hours in 45»l of ligase reaction mix after addition of 7.2 units of T₄ ligase. According to the Cohen's method (Proc. Natl. Acad. Sci. USA, 69, 2110-2114), the resulting mixture was used to transform E. coli JM103. Ampicillin-resistant colonies were selected and cultured to prepare plasmid DNA. About 1»g of the resultant plasmid was digested with restriction enzyme Bam HI (3 units) at 37°C for one hour in 30»l of a Bam HI reaction mix and analyzed by agarose gel electrophoresis. A plasmid which contained, downstream to Tac promoter, the gene encoding the insecticidal protein was selected and designated pKC6 (Fig.4).

### Step 4 Production of insecticidal protein by E. coli JM103/pKC6

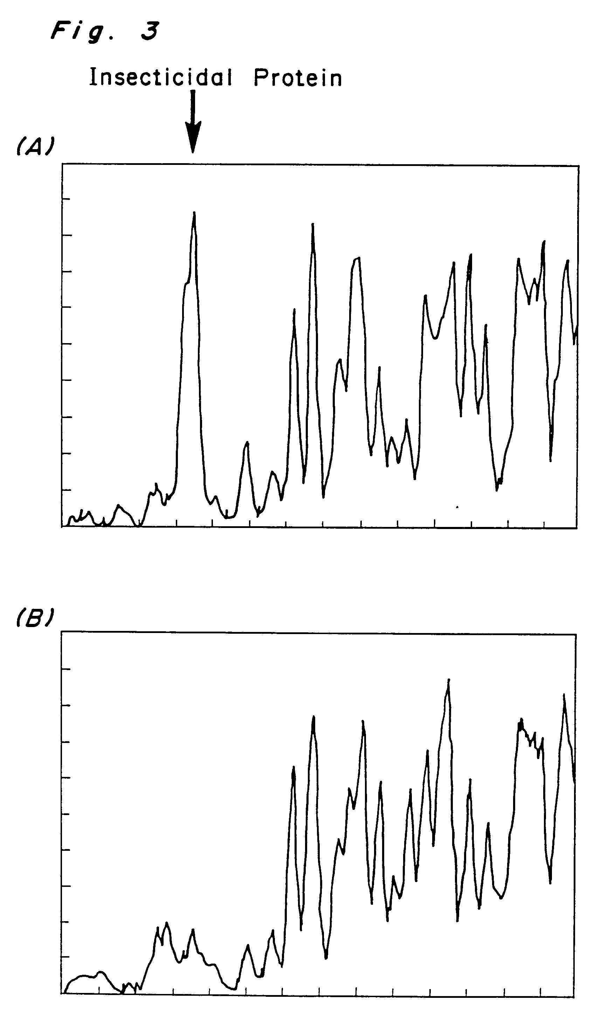

In substantial accordance with the procedure described in Example 1-C-(1), culture of E. coli JM103/pKC6 was subjected to SDS-polyacrylamide gel electrophoresis. A band containing insecticidal protein of 130KDal molecular weight was observed, which specifically cross-reacted with an antigen (IgG) to the insecticidal protein. Densitometeric determination of detectable bands on the gel revealed that the insecticidal protein produced by the strain amounted to 35% of the total proteins (Fig.3). Thus, it has been confirmed that E. coli JM103/pKC6 efficiently produces the insecticidal protein naturally produced by Bacillus thuringiensis aizawai a7A021. D. Evaluation of insecticidal protein produced in E. coli
The E. coli strain obtained above was cultured overnight in L broth liquid medium. The resultant culture (1.0ml) was transferred to 100ml of L broth liquid medium and cultured at 37°C for 16 hours. The cells were collected by centrifuging at 37°C for 10 minutes at 7,000 rpm (5000 g) and frozen at -80°C. Subsequently, the cells were frozen and then melted in room temperature. This manipulation was repeated three times. The cells were suspended in 20ml of TE buffer (10mM Tris-HCl, pH7.5, 1mM EDTA), and sonicated three times for 60 seconds. The crude suspension was centrifuged at 7,000 rpm (5000 g) for ten minutes, and the resultant precipitates were subjected to SDS-polyacrylamide gel electrophoresis, which revealed that about 80% of total proteins contained in the precipitates was the insecticidal protein.

The crude suspension of E. coli JM103/pKC6 obtained above killed nine larvae of Spodoptera litura among ten larvae tested at a concentration of 4mg/ml of the precipitates per mililiter of the suspension, and two among ten larvae of Plutella xylostella at a concentration of 2»g/ml.

The above experiment has shown that cultivation of E. coli JM103/pKC6 in an appropriate culture medium provides a significant amount of the insecticidal protein which is useful for combatting injurious insects such as Spodoptera litura and Plutella xylostella.

## Claims

1. A gene encoding an insecticidal protein having the amino acid sequence depicted in Figure 2.

2. The gene according to claim 1, which comprises the coding region of the DNA sequence depicted in Figure 2.

3. A gene which is related to a gene of claim 1 or 2 by insertions, deletions and/or substitutions of one or more nucleotides and which encodes an insecticidal protein having the biological activity of the insecticidal protein depicted in Figure 2.

4. The gene according to any one of claims 1 to 3, which is derived from Bacillus thuringiensis aizawai a7A021, said strain being obtainable by culturing Bacillus thuringiensis aizawai IPL No. 7 (FERM BP-1150) with acridine orange resulting in a loss of a 52 Md plasmid.

5. Plasmid pCA1 or pCA5 comprising the DNA sequence as depicted in Figure 2 inserted into the HincII site of plasmid PUC18, said plasmids having the restriction maps given in Figure 1 and containing said DNA sequence in different orientations.

6. An expression plasmid which comprises a gene according to any one of claims 1 to 4.

7. The expression plasmid according to claim 6, which is plasmid pKC6 (ATCC 67487).

8. A host microorganism containing a plasmid according to any one of claims 5 to 7.

9. The host microorganism according to claim 8, which is E. coli.

10. An insecticidal protein encoded by a gene of any one of claims 1 to 4.

11. A process for the preparation of an insecticidal protein according to claim 10 comprising culturing a host microorganism according to claim 8 or 9.

## Patentansprüche

1. Gen, das ein insektizides Protein mit der in Figur 2 dargestellten Aminosäuresequenz codiert.

2. Gen nach Anspruch 1, das den codierenden Bereich der in Figur 2 dargestellten DNA-Sequenz umfaßt.

3. Gen, das mit einem Gen von Anspruch 1 oder 2 verwandt ist durch Insertionen, Deletionen und/oder Substitutionen von einem oder mehreren Nucleotiden und das ein insektizides Protein mit der biologischen Aktivität des in Figur 2 dargestellten insektiziden Proteins codiert.

4. Gen nach einem der Ansprüche 1 bis 3, das abgeleitet ist von Bacillus thuringiensis aizawai a7A021, wobei der Stamm erhältlich ist durch Züchten von Bacillus thuringiensis aizawai IPL Nr. 7 (FERM BP-1150) mit Acridinorange, wodurch ein 52 MD-Plasmid verlorengeht.

5. Plasmid pCA1 oder pCA5, umfassend die in Figur 2 dargestellte DNA-Sequenz, inseriert in die HincII-Spaltstelle des Plasmids pUC18, wobei die Plasmide die in Figur 1 angegebenen Restriktionskarten haben und die genannte DNA-Sequenz in unterschiedlichen Orientierungen enthalten.

6. Expressionsplasmid, das ein Gen nach einem der Ansprüche 1 bis 4 umfaßt.

7. Expressionsplasmid nach Anspruch 6, das das Plasmid pKC6 (ATCC 67487) ist.

8. Wirtsmikroorganismus, der ein Plasmid nach einem der Ansprüche 5 bis 7 enthält.

9. Wirtsmikroorganismus nach Anspruch 8, der E. coli ist.

10. Insektizides Protein, das von einem Gen nach einem der Ansprüche 1 bis 4 codiert wird.

11. Verfahren zur Herstellung eines insektiziden Proteins nach Anspruch 10, umfassend das Züchten eines Wirtsmikroorganismus nach Anspruch 8 oder 9.

## Revendications

1. Gène codant pour une protéine insecticide ayant la séquence d'acides aminés représentée sur la figure 2.

2. Gène selon la revendication 1, qui comprend la région codante de la séquence d'ADN représentée sur la figure 2.

3. Gène qui s'apparente au gène de la revendication 1 ou 2 par des insertions, des délétions et/ou des substitutions d'un ou plusieurs nucléotides et qui code pour une protéine insecticide ayant l'activité biologique de la protéine insecticide représentée sur la figure 2.

4. Gène selon l'une quelconque des revendications 1 à 3, qui est dérivé Bacillus thuringiensis aizawai a7A021, ladite souche pouvant être obtenue par culture de Bacillus thuringiensis aizawai IPL No. 7 (FERM BP-1150) avec de l'acridine orange résultant en une perte d'un plasmide de 52 Md.

5. Plasmide pCA1 ou pCA5 comprenant la séquence d'ADN telle que représentée sur la figure 2, inséré dans le site HincII du plasmide pUC18, lesdits plasmides ayant les cartes de restriction données dans la figure 1 et contenant ladite séquence d'ADN dans différentes orientations.

6. Plasmide d'expression qui comprend un gène selon l'une quelconque des revendications 1 à 4.

7. Plasmide d'expression selon la revendication 6, qui est le plasmide pKC6 (ATCC 67487).

8. Micro-organisme hôte contenant un plasmide selon l'une quelconque des revendications 5 à 7.

9. Micro-organisme hôte selon la revendication 8, qui est E. coli.

10. Protéine insecticide codée par un gène de l'une quelconque des revendications 1 à 4.

11. Procédé pour la préparation d'une protéine insecticide selon la revendication 10 comprenant la culture d'un micro-organisme hôte selon la revendication 8 ou 9.
